# EUROPÄISCHE PATENTSCHRIFT

(11) **EP 2 396 054 B1**
(45) Veröffentlichungstag und Bekanntmachung des Hinweises auf die Patenterteilung: **17.05.2017**
(21) Anmeldenummer: 10704327.5
(22) Anmeldetag: 10.02.2010
(51) Int. Cl.: A61M 1/36

(54) **VERFAHREN ZUM ENTFERNEN VON BLUT AUS EINEM EXTRAKORPORALEN BLUTKREISLAUF FÜR EINE BEHANDLUNGSVORRICHTUNG NACH BEENDEN EINER BLUTBEHANDLUNGSSITZUNG UND VORRICHTUNG ZUM AUSFÜHREN DESSELBEN**
METHOD FOR WITHDRAWING BLOOD FROM AN EXTRACORPOREAL BLOOD CIRCULATION FOR A TREATMENT APPARATUS AFTER COMPLETING A BLOOD TREATMENT SESSION AND APPARATUS FOR PERFORMING THE SAME
PROCÉDÉ D'ÉLIMINATION DU SANG DANS LE CIRCUIT SANGUIN EXTRACORPOREL D'UN DISPOSITIF DE TRAITEMENT APRÈS LA FIN D'UNE SÉANCE DE TRAITEMENT SANGUIN, AINSI QUE DISPOSITIF DE MISE EN OEUVRE DE CE PROCÉDÉ

(30) Priorität: 11.02.2009 DE 102009008346
(43) Veröffentlichungstag der Anmeldung: 21.12.2011
(73) Patentinhaber: Fresenius Medical Care Deutschland GmbH, 61352 Bad Homburg (DE)
(72) Erfinder: GRONAU, Sören, 65428 Rüsselheim (DE); GÜNTHER, Götz, 61440 Oberursel (DE); HÄCKER, Jürgen, 61267 Neu-Anspach (DE)
(74) Vertreter: Bobbert & Partner Patentanwälte PartmbB
(86) Internationale Anmeldenummer: PCT/EP2010/000808
(87) Internationale Veröffentlichungsnummer: WO 2010/091841

(56) Entgegenhaltungen:
- WO-A1-96/40313
- WO-A1-2008/028579

## Beschreibung

Die vorliegende Erfindung betrifft ein Verfahren gemäß dem Oberbegriff des Anspruchs 1 zum Entfernen von Blut aus einem extrakorporalen Blutkreislauf für eine Behandlungsvorrichtung zur extrakorporalen Blutbehandlung eines Patienten nach Beenden der Blutbehandlungssitzung und eine Vorrichtung gemäß dem Oberbegriff des Anspruchs 11 zum Durchführen des erfindungsgemäßen Verfahrens.

Extrakorporale Blutkreisläufe sind üblicherweise EinwegArtikel und werden nach ihrer Verwendung entsorgt. Die Entsorgung ist kostenintensiv und nach Abfallgewicht zu vergüten. Aus diesem Grund, und auch zur Verringerung einer Kontaminationsgefahr, wird der Blutkreislauf daher vor seiner Entsorgung von Blut entleert.

Dazu ist aus der Praxis sowie aus der WO 01/51106 bekannt, das in einem extrakorporalen Blutkreislauf nach einer Blutbehandlungssitzung vorhandene Blut durch Einbringen von Luft in den extrakorporalen Blutkreislauf bzw. in sein Leitungsinneres zu entfernen. Diese Vorgehensweise birgt jedoch das Risiko, Luft in das Gefäßsystem des Patienten einzutragen, solange der extrakorporale Blutkreislauf mit dem Gefäßsystem des Patienten konnektiert ist. Ferner kann es bei diesem Verfahren zu einer Schaumbildung im Bereich eines im extrakorporalen Blutkreislauf vorhandenen Blutfilters kommen, was ein Entleeren des Bluts aus dem extrakorporalen Blutkreislauf erschwert. Im extrakorporalen Blutkreislauf verbleibendes Blut bildet aber wiederum ein Kontaminationsrisiko.

Aus der WO 96/40313 A1 sind Verfahren zur Desinfektion eines Blutschlauchsatzes bekannt.

Aus der WO 2008/028579 A1 und aus der DE 102 45 619 A1 sind Verfahren zum Entleeren eines Blutschlauchsatzes bekannt.

Es ist Aufgabe der vorliegenden Erfindung, ein weiteres Verfahren sowie eine geeignete Vorrichtung zum Entfernen von Blut aus einem extrakorporalen Blutkreislauf anzugeben.

Die erfindungsgemäße Aufgabe wird durch ein Verfahren mit den Merkmalen des Anspruchs 1 gelöst.

Das erfindungsgemäße Verfahren umfasst das Eintragen oder Einbringen von Luft und Substituatflüssigkeit in den extrakorporalen Blutkreislauf, beispielsweise nach Beenden einer Blutbehandlungssitzung.

Eine solche "Blutbehandlungssitzung" kann beispielsweise eine Behandlungseinheit mittels Hämodialyse, Hämofiltration, Hämodiafiltration und/oder eines Zellseparationsverfahrens und auf die Behandlung und/oder Reinigung von Blut gerichtet sein. Zum Durchführen einer solchen Blutbehandlung wird eine geeignete Blutbehandlungsvorrichtung verwendet.

Eine zum Durchführen des erfindungsgemäßen Verfahrens geeignete Blutbehandlungsvorrichtung weist einen extrakorporalen Blutkreislauf mit einem Leitungsinneren, verschiedene Fördereinrichtungen zum Einbringen und/oder Fördern verschiedener Fluide im Leitungsinneren des extrakorporalen Blutkreislaufs, und beispielsweise eine Einrichtung zum Behandeln des Bluts des Patienten, wie einen oder mehrere Blutfilter und/oder einen oder mehrere Dialysatoren und/oder einen oder mehrere Adsorber, auf. Sie kann ferner Behälter zum Aufbewahren von Fluiden, Elemente zum Einbringen der Fluide, wie beispielsweise Schlauchelemente und/oder Ventile, sowie weitere Einrichtungen, wie z.B. eine Luftabscheidekammer zum Entfernen von Luft aus dem Blut während der Blutbehandlung und/oder Sensoren und/oder Detektoren zum Erfassen verschiedener relevanter Parameter, wie beispielsweise einen Druck im extrakorporalen Blutkreislauf, aufweisen.

Ein "extrakorporaler Blutkreislaufs", wie im Folgenden erwähnt, kann beispielsweise als Schlauchsystem aus üblichem Kunststoffmaterial ausgestaltet sein und Einrichtungen aufweisen zum Verbinden und/oder Befestigen einzelner Abschnitte des extrakorporalen Blutkreislaufs mit bzw. an weiteren Einrichtungen der Blutbehandlungsvorrichtung und/oder dem Gefäßsystem eines Patienten, wie beispielsweise Klemmen, Zugangseinrichtungen zum Gefäßsystem des Patienten wie z.B. Konnektionsnadeln, Zuleitungseinrichtungen, über welche Medikamente in das Leitungsinnere eingebracht und/oder Proben des Leitungsinneren zu deren Untersuchung entnommen werden können, und dergleichen.

Der extrakorporale Blutkreislauf ist zum Aufnehmen und Fördern von Fluiden in seinem Leitungsinneren geeignet. In der vorliegenden Erfindung ist der extrakorporale Blutkreislauf insbesondere für das Aufnehmen und Fördern von Patientenblut sowie Luft und wenigstens einer Substituatflüssigkeit in seinem Leitungsinneren geeignet. Die im Leitungsinneren des extrakorporalen Blutkreislaufs jeweils vorhandenen Fluide geben den Inhalt des "Leitungsinneren des extrakorporalen Blutkreislaufs" zum entsprechenden Zeitpunkt an. Der Inhalt kann vorzugsweise gasförmig und/oder flüssig sein und ein oder mehrere Fluide umfassen.

Ferner können allgemein Flüssigkeiten, Gase, in Flüssigkeiten gelöste Gase und/oder Feststoffe, Suspensionen, Emulsionen, Dispersionen und weitere zum Zwecke der Blutbehandlung eines Patienten geeignete Zusammensetzungen den Inhalt des Leitungsinneren eines extrakorporalen Blutkreislaufs bilden oder Teil davon sein.

Fördereinrichtungen zum Einbringen und/oder Fördern von Fluiden innerhalb des Leitungsinneren des extrakorporalen Blutkreislaufs können entsprechend den eingesetzten Fluiden ausgewählt sein. Beispiele schließen Membranpumpen, Schlauchpumpen, Rollenpumpen usw. ein. Die "Blutpumpe" kann z.B. als Schlauchpumpe oder Rollenpumpe ausgeführt sein.

Als Fördereinrichtung zum Einbringen wenigstens einer Substituatflüssigkeit in das Leitungsinnere des extrakorporalen Blutkreislaufs und/oder Fördern eines Leitungsinhalts innerhalb des Leitungsinneren des extrakorporalen Blutkreislaufs kann eine peristaltische Pumpe, z.B. eine Rollenpumpe, eingesetzt werden. Es kann aber auch ein anderer Pumpentyp, z.B. eine Membranpumpe, insbesondere eine hochgenau dosierende Membranpumpe, eingesetzt werden.

Bei dieser Fördereinrichtung kann es sich um eine "zweite" Fördereinrichtung handeln, also eine Fördereinrichtung, welche nicht mit der Blutpumpe identisch ist. Die Blutpumpe kann jedoch auch derart ausgestaltet sein, dass sie sowohl die für eine Blutpumpe typische Funktion ausführt als auch die Funktion des Einbringens von Substituatflüssigkeit in das Leitungsinnere und/oder das Fördern von Leitungsinhalt erfüllt. Wenn im Folgenden allein der besseren Lesbarkeit wegen von einer "zweiten" Fördereinrichtung die Rede ist, so ist darunter die Blutpumpe oder eine sich hiervon unterscheidende Fördereinrichtung zu verstehen. Beide Varianten sind gleichermaßen von der vorliegenden Erfindung umfasst.

Auch kann die zweite Fördereinrichtung mit Zuleitungen zu verschiedenen Aufbewahrungseinrichtungen, wie Vorratsbehältern, welche wenigstens eine Substituatflüssigkeit enthalten können, ausgestaltet sein und/oder wenigstens eine Steuerungs- und/oder Regelungseinrichtung aufweisen, die ein automatisiertes Einbringen und/oder Fördern der wenigstens einen Substituatflüssigkeit erlaubt. Die zweite Fördereinrichtung kann als Rollenpumpe ausgestaltet sein.

Die zweite Fördereinrichtung und ihre Komponenten, insbesondere ihre Zuleitungen in den extrakorporalen Blutkreislauf, stellen vorzugsweise einen Teil des Schlauchsatzes für die extrakorporale Blutbehandlung dar und sind an oder in dem extrakorporalen Blutkreislauf vorgesehen, insbesondere mit diesem in geeigneter Weise, beispielsweise mit Hilfe von Klemmen, Anschlussstutzen oder dergleichen, verbunden.

Die zweite Fördereinrichtung kann sich alternativ auch innerhalb des extrakorporalen Blutkreislaufs befinden und Substituatflüssigkeit über eine Substituatleitung ansaugen.

Eine Substituatleitung ist vorzugsweise eine Zuleitung, die von einer Substituatquelle angeschlossen werden kann. Die Substituatleitung kann Bestandteil oder Zubehörteil eines Schlauchsatzes für die extrakorporale Blutbehandlung sein. Sie ist jedoch nicht unmittelbar Bestandteil des extrakorporalen Blutkreislaufs in dem Sinne, dass durch die Substituatleitung kein Blut strömt.

Das erfindungsgemäße Verfahren umfasst den Schritt des Einbringens oder Eintragens von Luft in das Leitungsinnere des extrakorporalen Blutkreislaufs durch Betreiben der Blutpumpe. Die Luft kann beispielsweise Atmosphärenluft sein. Die vorliegende Erfindung soll jedoch nicht auf die alleinige Verwendung von Luft beschränkt sein, sondern ferner alle, für die Zwecke der vorliegenden Erfindung geeigneten, gasförmigen Fluide anstelle von Luft einschließen.

Das "Eintragen von Luft in das Leitungsinnere des extrakorporalen Blutkreislaufs" nach Beenden der Blutbehandlungssitzung kann ausschließlich oder unterstützend durch die Blutpumpe erfolgen. So kann es z.B. auch möglich sein, Luft zusätzlich mit Hilfe geeigneter Einrichtungen in den extrakorporalen Blutkreislauf einzublasen und/oder einzusaugen oder durch Fluidkommunikations-Verbindungen mit der Umgebung, ohne maschinelles Zutun, eintreten zu lassen.

Es ist erfindungsgemäß auch möglich, Luft mittels der zweiten Fördereinrichtung zum Einbringen und/oder Fördern von Substituatflüssigkeit in das Leitungsinnere des extrakorporalen Blutkreislaufs einzubringen, insbesondere nachdem bereits Substituatflüssigkeit eingebracht wurde.

Kombinationen der vorgenannten Optionen sind erfindungsgemäß ebenfalls umfasst.

Das "Einbringen von Substituatflüssigkeit in das Leitungsinnere des extrakorporalen Blutkreislaufs" erfolgt durch Betreiben der zweiten Fördereinrichtung.

Die folgende Beschreibung soll ein Verständnis der der vorliegenden
Erfindung zugrunde liegenden Prinzipien und Funktionen der einzelnen Komponenten vereinfachen.

Eine "Substituatflüssigkeit" kann dabei beispielsweise jede zur Verwendung bei einer Blutbehandlung, wie z.B. einer Hämodiafiltration, allgemein bekannte Substituatflüssigkeit sein, vorzugsweise eine bereits während der Blutbehandlungssitzung verwendete und somit über eine Fluidverbindung bereits in den extrakorporalen Blutkreislauf eingebundene Lösung oder einbringbare isotone Kochsalzlösung, wie z.B. eine 0,9%-ige NaCl-Lösung.

Unter der Substituatflüssigkeit kann erfindungsgemäß auch ein anderes Fluid als eine Flüssigkeit verstanden werden, deren Verwendung sich erfindungsgemäß ebenfalls anbietet.

Unter der Substituatflüssigkeit kann beispielsweise auch ein z.B. mittels Perfusor über eine Zugabestelle eingebrachtes Medikament verstanden werden.

Obwohl die vorliegende Beschreibung an verschiedenen Stellen auf eine Behandlungsvorrichtung für eine Dialysebehandlung Bezug nimmt, ist die vorliegende Erfindung keinesfalls auf die Durchführung des Verfahrens zum Entleeren des extrakorporalen Blutkreislaufs nach einer Dialysebehandlung eingeschränkt. Vielmehr ist das erfindungsgemäße Verfahren zum Entfernen von Blut aus jedem extrakorporalen Blutkreislauf nach Beenden einer extrakorporalen Blutbehandlung geeignet.

Ein "Patient" im Sinne der vorliegenden Erfindung kann sowohl ein Mensch als auch ein Tier mit dem Bedarf nach einer extrakorporalen Blutbehandlung sein. Unter dem "Gefäßsystem des Patienten" ist hier der Blutkreislauf des Patienten als anatomische Struktur zu verstehen und umfasst u.a. *post partum* angelegte Fisteln, Shunts und dergleichen, ferner arterielle und venöse Leitungsstrukturen des Körpers und dergleichen.

Beim erfindungsgemäßen Verfahren werden sowohl Luft als auch Substituatflüssigkeit bzw. ein beliebiges, im Rahmen der vorliegenden Erfindung sinnvoll verwendbares Fluid in das Leitungsinnere des extrakorporalen Blutkreislaufs nach Beenden einer Blutbehandlungssitzung eingebracht oder eingetragen. Die Substituatflüssigkeit kann dabei unter anderem bevorzugt eine Spülfunktion des Leitungsinneren zum Vorbeugen eines Kontaminationsrisikos ausüben.

Sie kann bevorzugt eine Schaumbildung z.B. am Ausgang des Blutfilters verringern oder verhindern.

Die Luft kann unter anderem bevorzugt Flüssigkeiten wie Blut aus dem Leitungsinneren des extrakorporalen Blutkreislaufs verdrängen und somit das Gewicht des zu entsorgenden extrakorporalen Blutkreislaufs verringern.

Vorteilhafte Weiterentwicklungen der vorliegenden Erfindung sind jeweils Gegenstand der Unteransprüche.

In einer bevorzugten erfindungsgemäßen Ausführungsform umfasst das Erfassen einer qualitativen Änderung des Inhalts des Leitungsinneren des extrakorporalen Blutkreislaufs mit Hilfe wenigstens einer in einem Abschnitt des extrakorporalen Blutkreislaufs angeordneten Erfassungseinrichtung.

Die "qualitative Änderung" kann sich auf einen oder mehrere Bereiche oder Abschnitte des extrakorporalen Blutkreislaufs beziehen, beispielsweise einen Bereich oder Abschnitt, in welchem die Erfassungseinrichtung vorliegt.

Eine "qualitative Änderung des Inhalts des Leitungsinneren" schließt eine Änderung der Zusammensetzung des Inhalts des Leitungsinneren, wie beispielsweise eine Änderung der einzelnen Anteile an Blut, Substituatflüssigkeit und/oder Luft im Leitungsinneren oder einem Abschnitt hiervon, bezogen aufeinander, ein. Auch das Fehlen eines vormals vorhandenen Fluids kann eine Änderung der Zusammensetzung darstellen. Eine qualitative Änderung kann auch ein Übergang, z.B. von einem gasförmigen Inhalt zu einem flüssigen Inhalt, oder umgekehrt sein. Dies kann z.B. ein Übergang von Blut zu Luft sein. Ebenso kann eine qualitative Änderung ein Übergang von einem ersten flüssigen Inhalt zu einem zweiten, sich unterscheidenden flüssigen Inhalt sein, wie beispielsweise ein Übergang von Blut zu Substituatflüssigkeit. Solche Änderungen können z.B. leicht anhand einer optischen Änderung des Inhalts, wie einer Aufhellung oder einer Verdunkelung des Inhalts, erfasst werden.

Die in einem Abschnitt des extrakorporalen Blutkreislaufs angeordnete "Erfassungseinrichtung" kann z.B. ein optischer Sensor sein, der eine optische Änderung des Inhalts des Leitungsinneren oder eine Eigenschaft des Inhalts erfasst. So ist z.B. ein Blut-Luft-Inhalt im Leitungsinneren des extrakorporalen Blutkreislaufs aufgrund des vorhandenen Sauerstoffs heller als reines Blut. Weitere geeignete Sensoren schließen Drucksensoren zum Erfassen eines Druckabfalls bei einer Änderung des Inhalts im Leitungsinneren und Sensoren zum Erfassen einer Änderung der Dichte des Inhalts des Leitungsinneren des extrakorporalen Blutkreislaufs ein, ohne darauf beschränkt zu sein.

Der "Abschnitt des extrakorporalen Blutkreislaufs" kann ein arterieller und/oder venöser Abschnitt des extrakorporalen Blutkreislaufs sein. Der "arterielle Abschnitt" bezeichnet einen Abschnitt des extrakorporalen Blutkreislaufs, durch den das Blut aus dem Gefäßsystem des Patienten in Richtung zur Blutbehandlungseinrichtung strömt. Der "venöse Abschnitt" bezeichnet den Abschnitt des extrakorporalen Blutkreislaufs, durch den das Blut aus der Blutbehandlungseinrichtung zurück zum Gefäßsystem des Patienten strömt.

Die Erfassungseinrichtung kann Auskunft geben über die momentanen Verhältnisse im Leitungsinneren des extrakorporalen Blutkreislaufs an einem Abschnitt hiervon. Das erfindungsgemäße Verfahren kann somit besser und gezielter steuerbar bzw. regelbar sein. Ferner können auch mehrere Erfassungseinrichtungen zum Erfassen gleicher Parameter in verschiedenen Abschnitten des extrakorporalen Blutkreislaufs und/oder verschiedenen Erfassungseinrichtungen zum Erfassen verschiedener Parameter in gleichen Abschnitten des extrakorporalen Blutkreislaufs in selbigem angeordnet sein.

In einer ebenfalls bevorzugten Weiterbildung des erfindungsgemäßen Verfahrens umfasst der extrakorporale Blutkreislauf wenigstens eine mit einem Abschnitt des Gefäßsystems des Patienten konnektierbare Zugangseinrichtung, und das Verfahren umfasst ein Dekonnektieren des extrakorporalen Blutkreislaufs vom Gefäßsystem des Patienten, insbesondere im Bereich einer ersten, beispielsweise arteriellen, Zugangseinrichtung, insbesondere an einem Ende des extrakorporalen Blutkreislaufs.

Ein solcher "Abschnitt des Gefäßsystems" kann, wie oben, ein arterieller und/oder venöser Abschnitt des extrakorporalen Blutkreislaufs sein. Teil eines solchen Abschnitts kann eine "mit dem Gefäßsystem des Patienten konnektierbare Zugangseinrichtung" sein, die als Kanüle, Nadel, Katheter oder dergleichen ausgebildet und bei Bedarf mit dem Gefäßsystem des Patienten konnektiert werden kann. Eine solche Zugangseinrichtung kann als so genannte DoubleNeedle- oder SingleNeedle-Variante ausgeführt sein.

Während bei der DoubleNeedle-Variante jeweils eine arterielle Konnektionsnadel und eine venöse Konnektionsnadel mit dem Gefäßsystem des Patienten und mit dem extrakorporalen Blutkreislauf bzw. dessen arteriellen und venösen Abschnitt konnektiert sind, weist die SingleNeedle lediglich eine mit dem Gefäßsystem des Patienten direkt verbundene Konnektionsnadel und einen sich anschließend "Y"-förmig aufgabelnden Leitungsabschnitt auf, dessen "Schenkel" sich jeweils zu dem arteriellen und dem venösen Abschnitt des extrakorporalen Abschnitts verzweigen.

Das "Dekonnektieren des extrakorporalen Blutkreislaufs vom Gefäßsystem des Patienten" meint das Unterbrechen einer Verbindung zwischen dem extrakorporalen Blutkreislauf und dem Gefäßsystem des Patienten in einem Abschnitt des extrakorporalen Blutkreislaufs, beispielsweise an einem Ende hiervon. Dabei kann die Unterbrechung sowohl am arteriellen als auch am venösen Abschnitt erfolgen, wobei in der vorliegenden Erfindung ein Dekonnektieren des arteriellen Abschnitts des extrakorporalen Blutkreislaufs bevorzugt ist.

Unter einem Dekonnektieren im "Bereich der ersten Zugangseinrichtung" kann z.B. das Herausziehen der arteriellen Konnektionsnadel eines DoubleNeedle-Zugangs verstanden werden.

Unter einem Dekonnektieren kann auch das Unterbrechen der Strömungsverbindung zwischen dem arteriellen Abschnitt des extrakorporalen Blutkreislaufs und der arteriellen Konnektionsnadel verstanden werden.

Bei der SingleNeedle-Variante kann das Unterbrechen der Verbindung zwischen dem arteriellen Schenkel des "Y"-förmigen Abschnitts des extrakorporalen Blutkreislaufs und der einzigen mit dem Gefäßsystem des Patienten verbundenen Konnektionsnadel verstanden werden. Das offene Lumen des arteriellen Schenkels des Y-Stücks kann nach seinem Abtrennen auf beliebige Weise (manuell, maschinell, automatisch, usw.) verschlossen werden.

Alternativ oder zusätzlich kann gleiches auch für den venösen Abschnitt des extrakorporalen Blutkreislaufs und den venösen Zugang zum Gefäßsystem des Patienten angewandt werden.

Die Auswahl der Zugangseinrichtung mit geeignetem Zugang zum Gefäßsystem des Patienten ist für das Ausführen der vorliegenden Erfindung nicht entscheidend. Der Einfachheit halber wird hier des Öfteren auf den DoubleNeedle-Zugang verwiesen, ohne die vorliegende Erfindung darauf einschränken zu wollen. Es ist zu beachten, dass die Erfindung in gleicher Weise mit einem SingleNeedle-Zugang oder jeder weiteren, zum Zwecke einer Blutbehandlung geeigneten Zugangseinrichtung ausführbar ist.

Das Dekonnektieren des extrakorporalen Blutkreislaufs ermöglicht auf einfache und unkomplizierte Weise das Eintragen oder Einbringen von Luft in das Leitungsinnere des extrakorporalen Blutkreislaufs zum Ausführen des erfindungsgemäßen Verfahrens durch eine beim Dekonnektieren hergestellte Fluidkommunikation mit der Atmosphäre.

Eine weiter bevorzugte Ausführungsform der vorliegenden Erfindung schließt das Fördern eines definierten Volumens an Luft durch die Blutpumpe ein.

Um ein "definiertes Volumen an Luft" zu fördern, kann die Blutpumpe beispielsweise für eine vorher festgelegte Zeitdauer und/oder eine bestimmte Anzahl an Umdrehungen betrieben werden.

Das "Fördern eines definierten Volumens an Luft" kann aber auch dem Fördern eines bestimmen Fördervolumens und/oder dem Fördern über eine vorbestimmte Förderstrecke des Inhalts entlang des Leitungsinneren des extrakorporalen Blutkreislaufs entsprechen.

Eine weiter bevorzugte Ausführungsform des erfindungsgemäßen Verfahrens umfasst das Fördern des "Luft-Blut-Inhalts" im Leitungsinneren des extrakorporalen Blutkreislaufs, bis der "Luft-Blut-Inhalt" eine Zugabestelle des extrakorporalen Blutkreislaufs für Substituatflüssigkeit in das Leitungsinnere des extrakorporalen Blutkreislaufs erreicht.

Der "Luft-Blut-Inhalt" kann z.B. in Richtung weg vom dekonnektierten Ende des extrakorporalen Blutkreislaufs gefördert werden.

Der "Luft-Blut-Inhalt" bezeichnet den Inhalt, der durch Einbringen von Luft in den nach Beenden der Blutbehandlungssitzung bereits Blut enthaltenden extrakorporalen Blutkreislauf erzeugt wird, wobei Blut und Luft die einzigen oder im Wesentlichen die einzigen Fluide sein können, die im Inneren des extrakorporalen Blutkreislauf vorliegen.

Eine "Zugabestelle des extrakorporalen Blutkreislaufs für Substituatflüssigkeit in das Leitungsinnere des extrakorporalen Blutkreislaufs" kann in dem arteriellen und/oder dem venösen Abschnitt des extrakorporalen Blutkreislaufs angeordnet sein. Bevorzugt ist die "Zugabestelle" in einem Abschnitt des extrakorporalen Blutkreislaufs angeordnet, den das Blut in Richtung weg vom dekonnektierten Ende des extrakorporalen Blutkreislaufs durchströmt. Besonders bevorzugt ist die Zugabestelle in dem Abschnitt des extrakorporalen Blutkreislaufs angeordnet, den das Blut vor Erreichen und Durchströmen der Blutbehandlungseinrichtung, wie beispielsweise eines Blutfilters, durchströmt. Eine solche Zugabestelle kann in geeigneter Weise zum Zugeben von Substituatflüssigkeit in den extrakorporalen Blutkreislauf ausgewählt sein, derart, dass Substituatflüssigkeit als eine Art Trennschicht oder als eine Art Trennvolumen zwischen das Blutvolumen und das Luftvolumen mittels der zweiten Fördereinrichtung eingebracht werden kann.

Geeignete Beispiele für eine Zugabestelle schließen ein Öffnungs-/Verschlussventil, einen Absperrhahn, eine zuschaltbare Nebenleitung eines verzweigten Abschnitts des extrakorporalen Blutkreislaufs usw. ein.

Das "Fördern des Luft-Blut-Inhalts" kann durch Betreiben der Blutpumpe erfolgen. Wenn der Luft-Blut-Inhalt bzw. eine sich ausbildende Luft-Blut-Grenze oder Luft-Blut-Übergangs- bzw. Mischbereich die Zugabestelle des extrakorporalen Blutkreislaufs für Substituatflüssigkeit in das Leitungsinnere des extrakorporalen Blutkreislaufs erreicht, kann das Fördern des Luft-Blut-Inhalts gestoppt werden. Dies kann durch Stoppen der Blutpumpe erreicht werden. Das exakte Stoppen des Förderns des Luft-Blut-Inhalts an oder eine vorgegebene Distanz vor der Zugabestelle für Substituatflüssigkeit kann durch das Einbringen eines definierten Volumens an Luft erreicht werden.

Unter dem "Erreichen" der Zugabestelle ist erfindungsgemäß vorzugsweise und beispielhaft zu verstehen, dass der Luft-Blut-Inhalt bis zu einer Position in Nähe oder in unmittelbarer Nähe der Zugabestelle (Mündung, Einstrombereich oder dgl.) für die Substituatflüssigkeit - z.B. davor - oder bis exakt an die Position der Zugabestelle für Substituatflüssigkeit gefördert wird. Der Begriff "Erreichen" soll daher nicht auf ein absolutes "Ankommen" des Luft-Blut-Inhalts an der Zugabestelle für Substituatflüssigkeit beschränkt sein. Vielmehr soll er eine relative oder anderweitig geeignete Position des geförderten Leitungsinneren bezüglich der Zugabestelle für Substituatflüssigkeit definieren. Diese Definition soll in ihrer Breite ferner für alle hier angegebenen Ausführungsformen gelten.

So kann in einer bevorzugten Ausführungsform die Blutpumpe angehalten werden, wenn die Luft-Blut-Grenze die Zugabestelle noch nicht erreicht hat, also davor. Dies kann vorteilhaft vorzugsweise derart erfolgen, dass keine Luft in einen Blutfilter gefördert wird.

Von der Erfindung umfasst ist auch eine Anordnung, bei welcher das Fördern von Substituatflüssigkeit beginnt, noch während die Blutpumpe Luft ansaugt, die Luft die Zugabestelle für die Substituatflüssigkeit aber noch nicht erreicht hat. Auf diese Weise kann vorteilhaft ein "Freispülen" des Blutfilters erfolgen.

Daneben kann zusätzlich eine weitere Erfassungseinrichtung, wie beispielsweise ein geeignet ausgebildeter Luft-Blutdetektor, in dem Abschnitt des extrakorporalen Kreislaufs, wie beispielsweise dem arteriellen Abschnitt des extrakorporalen Blutkreislaufs, z.B. zwischen der Blutpumpe und der Zugabestelle für Substituatflüssigkeit, angeordnet sein und das Auftreten von Luft im Leitungsinneren des extrakorporalen Blutkreislaufs erfassen. Ein solcher Luft-Blutdetektor kann wiederum als optischer Sensor ausgebildet sein und das Auftreten von Luft im Leitungsinneren des extrakorporalen Blutkreislaufs als optische Änderung des Leitungsinhalts erfassen.

Wenn der Luft-Blut-Inhalt die Zugabestelle für Substituatflüssigkeit erreicht und/oder eine Erfassungseinrichtung das Auftreten von Luft im Leitungsinneren des extrakorporalen Blutkreislaufs erfasst, wird durch Betreiben der zweiten Fördereinrichtung, zum Beispiel einer Schlauch- oder Rollenpumpe, Substituatflüssigkeit an der Zugabestelle für Substituatflüssigkeit in das Leitungsinnere des extrakorporalen Blutkreislaufs eingetragen oder eingebracht.

Die Förderung von Substituatflüssigkeit kann vorzugsweise bereits beginnen, bevor die Blutpumpe Luft ansaugt. Alternativ dazu kann sie jedoch vorzugsweise auch beginnen, während die Blutpumpe Luft ansaugt.

Bei der in das Leitungsinnere des extrakorporalen Blutkreislaufs eingetragenen oder eingebrachten Substituatflüssigkeit kann es sich um eine unbestimmte Menge oder um eine vorbestimmte Menge oder um eine begrenzte Menge an Substituatflüssigkeit handeln.

Das "Einbringen einer vorbestimmten Substituatflüssigkeitsmenge" kann einen "Luft-Substituatflüssigkeit-Blut-Inhalt" erzeugen. Der Begriff "Luft-Substituatflüssigkeit-Blut-Inhalt" bezeichnet hierbei, dass die Substituatflüssigkeit zwischen das durch den extrakorporalen Blutkreislauf geförderte Blut und die durch den extrakorporalen Blutkreislauf geförderte Luft eingebracht wird und so eine Art "Puffer" zwischen Luft und Blut bildet.

Eine "vorbestimmte Substituatflüssigkeitsmenge" kann einem bestimmten Fördervolumen und/oder einer bestimmten Förderstrecke des Inhalts entlang des Leitungsinneren des extrakorporalen Blutkreislaufs entsprechen und beispielsweise durch Betreiben einer hochgenau dosierenden Membranpumpe erfolgen.

Die Substituatflüssigkeitsmenge kann vorzugsweise als Größe, zum Beispiel als Volumen mit vorgegebener Maßzahl und Einheit, vorbestimmt sein. Die absolute Größe der Substituatflüssigkeitsmenge kann vorzugsweise beispielsweise in einer Steuereinrichtung der erfindungsgemäßen Vorrichtung hinterlegt und/oder eingebbar sein. Die Substituatflüssigkeitsmenge kann vorzugsweise im Rahmen der technischen Genauigkeit exakt gefördert werden.

Um eine exakte Menge an Substituatflüssigkeit vorzubestimmen, können zum Beispiel technische Spezifikationen des eingesetzten extrakorporalen Blutkreislaufs, wie beispielsweise die Innenvolumina des Schlauchsatzes, in der Steuereinrichtung gespeichert oder eingegeben werden. Anhand der technischen Spezifikationen der einzelnen Komponenten des extrakorporalen Blutkreislaufs können zum Beispiel eine erforderliche Förderzeit und/oder ein Fördervolumen berechnet werden.

Eine "begrenzte Substituatflüssigkeitsmenge" kann eine, zum Beispiel anhand von Erfahrungswerten des Bedienpersonals ausgewählte, Menge an Substituatflüssigkeit sein. Vorzugsweise kann eine begrenzte Menge an Substituatflüssigkeit eingebracht und so lange gefördert werden, bis an einer weiteren Erfassungseinrichtung das Auftreten von Substituatflüssigkeit im Leitungsinneren des extrakorporalen Blutkreislaufs erfasst wird. Eine begrenzte Substituatflüssigkeitsmenge muss daher nicht genau bekannt sein und/oder einem bestimmten Fördervolumen entsprechen. Eine begrenzte Substituatflüssigkeitsmenge kann jedoch indirekt durch das Innenvolumen der von der Substituatflüssigkeitsmenge durchströmten Komponenten des extrakorporalen Blutkreislaufs, insbesondere das Innenvolumen des Abschnitts von der Zugabestelle für Substituatflüssigkeit und/oder der Blutbehandlungseinrichtung bis zu einer weiteren Erfassungseinrichtung und das Erkennen der Substituatflüssigkeit durch diese mit Beenden der Zufuhr weiterer Substituatflüssigkeit, bestimmt im Sinne von begrenzt sein. Das Volumen ist dabei somit bestimmt im Sinne von "begrenzt" ohne jedoch exakt bekannt zu sein, und ohne beispielsweise in Milliliter angegeben werden zu können und/oder ohne in einer Steuerung hinterlegt worden oder eingebbar zu sein. Das Einbringen einer begrenzten Substituatflüssigkeitsmenge kann zum Beispiel von Vorteil sein, wenn der Filtertyp einer Blutbehandlungsvorrichtung oder dessen Fassungsvermögens nicht bekannt oder nicht richtig angegeben ist.

Dabei kann die Substituatflüssigkeit aus einem dafür vorgesehenen Vorratsbehälter über entsprechende Leitungssysteme des extrakorporalen Blutkreislaufs an der Zugabestelle für die Substituatflüssigkeit in den extrakorporalen Blutkreislauf eingebracht werden.

Der auf diese Weise erzeugte "Luft-Substituatflüssigkeit-Blut-Inhalt" kann in Richtung weg vom dekonnektierten Ende durch Betreiben der Blutpumpe und/oder durch Betreiben der zweiten Fördereinrichtung gefördert werden.

Nach Einbringen von Substituatflüssigkeit und somit Erzeugen des "Luft-Substituatflüssigkeit-Blut-Inhalts" kann ferner weiter Luft in das Leitungsinnere des extrakorporalen Blutkreislaufs eingebracht werden. Dies kann insbesondere durch Betreiben der Blutpumpe und/oder durch Betreiben der zweiten Fördereinrichtung erfolgen. Das Ansaugen und Einbringen von Luft durch die zweite Fördereinrichtung kann zum Beispiel durch Herstellen einer Verbindung zwischen der Saugseite der zweiten Fördereinrichtung und einem Äußeren des extrakorporalen Blutkreislaufs, z.B. der Atmosphäre, erfolgen.

Das weitere Einbringen von Luft kann insbesondere dazu dienen, das Leitungsinnere des extrakorporalen Blutkreislaufs im Wesentlichen von im Inneren vorhandenen Fluiden, insbesondere Blut und Substituatflüssigkeit, zu befreien oder die hiervon im Leitungsinneren vorliegenden Mengen zu verringern.

Das Fördern des "Luft-Substituatflüssigkeit-Blut-Inhalts" kann beispielsweise dann gestoppt werden, wenn die Erfassungseinrichtung Substituatflüssigkeit im Leitungsinneren des extrakorporalen Blutkreislaufs erkennt.

Die "Erfassungseinrichtung" ist wie vorstehend definiert und kann beispielsweise im venösen Abschnitt des extrakorporalen Blutkreislaufs, z.B. zwischen der Blutbehandlungseinrichtung und der venösen Zugangseinrichtung zum Gefäßsystem des Patienten und insbesondere zwischen einer Tropfkammer im venösen Abschnitt und der venösen Zugangseinrichtung, angeordnet sein.

Die Erfassungseinrichtung kann das Auftreten von Substituatflüssigkeit in einem bestimmten Abschnitt des Leitungsinneren des extrakorporalen Blutkreislaufs beispielsweise anhand einer optischen Änderung des Inhalts des Leitungsinneren erkennen.

Wenn die Erfassungseinrichtung das Auftreten von Luft oder Substituatflüssigkeit in dem Leitungsinneren des extrakorporalen Blutkreislaufs erkennt, kann das Fördern des "Luft-Substituatflüssigkeit-Blut-Inhalts" gestoppt werden.

Dies kann durch Stoppen der zweiten Fördereinrichtung erfolgen.

Ferner ist in einer weiteren Ausführungsform des erfindungsgemäßen Verfahrens bevorzugt, die Erfassungseinrichtung mit vorgegebener Distanz zu einer zweiten Zugangseinrichtung anzuordnen und den Inhalt des Leitungsinneren über die vorgegebene Distanz bis zur Zugangseinrichtung zu fördern, nachdem Luft an der Erfassungseinrichtung erkannt wurde.

Die "zweite Zugangseinrichtung" kann, ohne darauf beschränkt zu sein, eine venöse Konnektionsnadel eines DoubleNeedle-Zugangs oder ein "venöser" Schenkel eines Y-Stückes sein.

Wenn von der Erfassungseinrichtung Luft erkannt wurde, kann der "Luft-Substituatflüssigkeit-Blut-Inhalt" über die vorgegebene Distanz bis zur zweiten Zugangseinrichtung gefördert werden und so Blut und gegebenenfalls auch Substituatflüssigkeit aus dem extrakorporalen Blutkreislauf entfernt werden. Dabei kann das Blut lediglich aus dem extrakorporalen Blutkreislauf entleert oder zugleich in das Gefäßsystem des Patienten rückgeführt werden.

In einer weiter bevorzugten Ausführungsform des erfindungsgemäßen Verfahrens wird das im Leitungsinneren des extrakorporalen Blutkreislaufs enthaltene Blut - insbesondere im Wesentlichen vollständig - über die zweite Zugangseinrichtung in das Gefäßsystem des Patienten rückgeführt. Der Begriff "im Wesentlichen vollständig rückgeführt" meint hierbei, dass das im Leitungsinneren des extrakorporalen Blutkreislaufs vorhandene Blut nahezu rückstandsfrei aus dem extrakorporalen Blutkreislauf entfernt wird. Die gegebenenfalls im extrakorporalen Blutkreislauf aus technischen Gründen wie Benetzungsverhalten oder Blut-Restbestände in der Tropfkammer verbleibenden Rückstände sind dabei als vernachlässigbar gering anzusehen.

Das "Rückführen von Blut in das Gefäßsystem des Patienten" kann erfolgen, wenn ein Ende des extrakorporalen Blutkreislaufs, wie beispielsweise das Ende des venösen Abschnitts, z.B. die venöse Konnektionsnadel, mit dem Gefäßsystem des Patienten konnektiert ist. Diese Verbindung kann nach Beenden der Blutbehandlungssitzung beibehalten sein oder erneut hergestellt werden.

Daneben kann es weiter bevorzugt sein, auch die Substituatflüssigkeit, insbesondere im Wesentlichen vollständig, aus dem Leitungsinneren des extrakorporalen Blutkreislaufs zu entfernen. Dies kann durch Fördern des Inhalts des Leitungsinneren des extrakorporalen Blutkreislaufs lediglich über die vorgegebene Distanz zwischen der Erfassungseinrichtung und der Zugangseinrichtung erreicht werden, nachdem die Erfassungseinrichtung das Auftreten von Luft erkannt hat. Die vorgegebene Distanz kann mit dem durch die Blutpumpe eingetragenen definierten Fördervolumen an Luft korrelieren.

Das "im Wesentlichen vollständige Entfernen" der Substituatflüssigkeit aus dem Leitungsinneren des extrakorporalen Blutkreislaufs kann, wie vorstehend in Bezug auf das im Leitungsinneren enthaltene Blut beschrieben, als ebenfalls nahezu rückstandsfrei angesehen werden.

Ein vollständiges oder nahezu vollständiges Entfernen ist erfindungsgemäß jedoch nicht erforderlich, um Vorteile zu erzielen.

Nach dem kompletten Dekonnektieren des Patienten (arteriell und venös) könnte nach der Blutrückgabe an den Patienten optional noch eines der bekannten (halb-)automatisierten Verfahren zum mehr oder weniger kompletten Entleeren des Blutschlauchsatzes durchgeführt werden. Solche Verfahren fördern Flüssigkeitsreste z.B. in den sog. Rinse-Port der Dialysemaschine. Natürlich kann die restliche Flüssigkeit in jeden beliebigen Ablauf gefördert werden. Derartige Entleerungsverfahren können sich an das erfindungsgemäße Verfahren anschließen bzw. Teil einer Ausführungsform desselben sein. Sie sind jedoch zur Ausführung der Erfindung nicht erforderlich.

Die erfindungsgemäße Aufgabe wird ferner durch eine Vorrichtung mit den Merkmalen des Anspruchs 11 gelöst. Eine solche Vorrichtung umfasst wenigstens einen extrakorporalen Blutkreislauf mit einem Leitungsinneren, wenigstens eine an oder in dem extrakorporalen Blutkreislauf angeordnete Blutpumpe zum Fördern von Blut innerhalb des Leitungsinneren des extrakorporalen Blutkreislaufs, wenigstens eine mit Verbindung zum extrakorporalen Blutkreislauf vorgesehene - ggf. zusätzliche - zweite Fördereinrichtung zum Einbringen wenigstens einer Substituatflüssigkeit in das Leitungsinnere und/oder zum Fördern eines Leitungsinhalts innerhalb des Leitungsinneren des extrakorporalen Blutkreislaufs, und wenigstens eine Steuerungs- oder Regelungseinrichtung, mit der ein Verfahren gemäß der vorliegenden Erfindung durchführbar ist.

Eine solche Steuerungs- oder Regelungseinrichtung kann entsprechend zum Durchführen eines erfindungsgemäßen Verfahrens ausgestaltet und/oder konfiguriert sein. Sie kann gegebenenfalls weitere Einrichtungen, wie beispielsweise Speichereinrichtungen, Zugabeeinrichtungen, automatisierte Signalgebungseinrichtungen usw. umfassen.

Da mit der erfindungsgemäßen Vorrichtung das vorstehend beschriebene erfindungsgemäße Verfahren durchführbar ist, wird zur Vermeidung von Wiederholungen auf die vorstehend beschriebenen Ausführungen derselben verwiesen.

Eine Weiterbildung der erfindungsgemäßen Vorrichtung sieht das Anordnen wenigstens einer Erfassungseinrichtung zum Erfassen wenigstens einer Änderung des Inhalts des Leitungsinneren des extrakorporalen Blutkreislaufs oder einer Eigenschaft des Inhalts in einem Abschnitt des extrakorporalen Blutkreislaufs vor. Bei einer Eigenschaft des Inhalts kann es sich um eine Zusammensetzung, eine physikalische, chemische oder biologische Größe, beispielsweise eine Transparenz, einen pH-Wert und vieles dergleichen mehr handeln. Eine derartige Erfassungseinrichtung kann der vorstehend beschriebenen entsprechen, so dass zur Vermeidung von Wiederholungen auf deren obige Beschreibung verwiesen wird. Umgekehrt kann das zur zweiten Erfassungseinrichtung Ausgeführte ungeschmälert auch auf jede andere hier mitoffenbarte Erfassungseinrichtung zutreffen.

Die erfindungsgemäße Vorrichtung kann weitere, zur Durchführung des erfindungsgemäßen Verfahrens geeignete Einrichtungen und/oder weitere Einrichtungen zur Durchführung einer Blutbehandlung, wie beispielsweise eine Blutbehandlungseinrichtung, z.B. einen Blutfilter, eine Alarmeinrichtung, "Not-Aus"-Einrichtungen und dergleichen mehr umfassen.

Jede der Einrichtungen kann sowohl in dem extrakorporalen Blutkreislauf angeordnet als auch mit diesem verbunden sein. Die zu diesem Zweck geeigneten Zuleitungen und/oder Anschlüsse, Befestigungseinrichtungen und dergleichen können von der Vorrichtung der vorliegenden Erfindung ebenfalls erfasst sein.

Eine Vorrichtung gemäß der vorliegenden Erfindung kann, ohne darauf beschränkt zu sein, zum Durchführen einer Hämodialyse, einer Hämofiltration, einer Hämodiafiltration und Separationsverfahren geeignet sein.

Es wird darauf hingewiesen, dass die vorliegende Erfindung nicht auf die vorstehend beschriebenen Ausführungsformen der erfindungsgemäßen Vorrichtung beschränkt ist. Ferner sind alle weiteren Behandlungsvorrichtungen von der vorliegenden Erfindung eingeschlossen, mit denen das erfindungsgemäße Verfahren ebenfalls durchführbar ist.

Die vorliegende Erfindung kann in vorteilhafter Weise zum Entfernen von Blut aus dem extrakorporalen Blutkreislauf für eine Behandlungsvorrichtung zur extrakorporalen Blutbehandlung eines Patienten nach Beenden einer Blutbehandlungssitzung eingesetzt werden: Da sich zu gleicher Zeit Luft und Substituatflüssigkeit in Form eines Luft-Substituatflüssigkeit-Blut-Inhalts im Leitungsinneren des extrakorporalen Blutkreislaufs nach Beenden der Blutbehandlungssitzung befinden, kann das im Leitungsinneren des extrakorporalen Blutkreislaufs vorhandene Blut aus dem extrakorporalen Blutkreislauf entfernt werden. Dies kann ohne Gefahr erfolgen, Luft in den Körper des Patienten einzubringen.

Daneben kann einem Kontaminationsrisiko bei der weiteren Handhabung oder der Entsorgung des extrakorporalen Blutkreislaufs vorgebeugt werden.

Ferner kann das Brutto-Abfallgewicht des extrakorporalen Blutkreislaufs aus Blutkreislaufmaterial und flüssigem Blutkreislaufinhalt nach dessen Verwendung in einer Blutbehandlungssitzung verringert werden. Da eine Entsorgung des extrakorporalen Blutkreislaufs nach Gewicht berechnet wird, können somit in vorteilhafter Weise Kosten eingespart werden.

Durch Fördern, insbesondere volumendefinierter, Mengen an Luft und Substituatflüssigkeit kann der Inhalt des Leitungsinneren des extrakorporalen Blutkreislaufs über vorgegebene Distanzen im extrakorporalen Blutkreislauf gefördert werden und so ein wesentliches Entfernen von Flüssigkeiten (Blut und ggf. auch Substituatflüssigkeit) aus dem Leitungsinneren des extrakorporalen Blutkreislaufs gewährleistet werden. Dabei bedarf es nur einer geringen Menge an Substituatflüssigkeit. Dies senkt vorteilhaft die Kosten und den Bereitstellungsaufwand für das verwendete Substituat.

Die im Blutschlauchsatz gegebenenfalls zurückbleibende Menge an Substituatflüssigkeit kann beispielsweise dem Volumen des venösen Abschnitts des extrakorporalen Blutkreislaufs entsprechen und beispielsweise nur etwa 30 ml betragen. Damit wird bereits in vorteilhafter Weise zur Verringerung des Abfallgewichts des extrakorporalen Blutkreislaufs beigetragen. Die im Blutschlauchsatz verbleibende - hinsichtlich ihres Beitrags zum Gesamtgewicht des Schlauchsatzes vernachlässigbare - Substituatflüssigkeitsmenge kann in vorteilhafter Weise als Sicherheitspuffer dienen und zu diesem Zweck im Blutschlauchsatz verbleiben. Auf diese Weise kann es ein Einbringen von Luft in den Blutkreislauf des Patienten verhindern.

Das Einbringen von Substituatflüssigkeit zwischen den Luft-Blut-Inhalt im Leitungsinneren kann in vorteilhafter Weise dazu beitragen, das Eintragen von Luft in das Gefäßsystem des Patienten zu vermeiden bei Entleeren des extrakorporalen Blutkreislaufs bei dessen Konnektion mit dem Patienten, und dennoch können mittels der Substituatflüssigkeit als Spülflüssigkeit Blutrückstände aus dem Leitungsinneren des extrakorporalen Blutkreislaufs gespült und so ein Kontaminationsrisiko weiter ausgeschlossen werden. Der extrakorporale Blutkreislauf kann somit sicher und mit geringem Rest-Gesamtgewicht bzw. Brutto-Abfallgewicht entsorgt werden.

Das Einbringen von Substituatflüssigkeit kann ferner dazu beitragen, einen Blutfilter mit verschiedenen Kapillarinnendurchmessern von Blut zu reinigen, was durch ledigliches Durchströmen mit Luft aufgrund des geringeren Druckabfalls über den bereits luftgefüllten Kapillaren im Gegensatz zu den noch mit Blut gefüllten Kapillaren technisch schlecht realisierbar wäre.

Daneben kann es auch möglich sein, eine Schaumbildung z.B. am Ausgang des Blutfilters zu minimieren, indem dort Substituatflüssigkeit, also insbesondere eine Flüssigkeit, nicht aber allein ein Gas (z.B. Luft) zum Verdrängen von Blut oder Entleeren eingesetzt wird.

Da das erfindungsgemäße Verfahren direkt nach Beenden einer Blutbehandlungssitzung durchgeführt werden kann, ist es einfach und leicht durchzuführen und erfordert keine technisch aufwändigen, zeit- und/oder kostenintensiven Schritte.

Das erfindungsgemäße Verfahren kann in vorteilhafter Weise mit der bei einer Blutbehandlung ohnehin eingesetzten oder vorliegenden Substituatflüssigkeit, wie beispielsweise einer isotonen Kochsalzlösung, z.B. einer 0,9%-igen NaCl-Lösung, durchgeführt werden. Dies trägt wiederum in vorteilhafter Weise zu einer Kosten- und Zeitersparnis bei.

Ferner kann das erfindungsgemäße Verfahren ein Entfernen von Blut aus dem arteriellen Abschnitt des extrakorporalen Blutkreislaufs und insbesondere aus der arteriellen Konnektionsnadel und ein Rückführen desselben in das Gefäßsystem des Patienten ermöglichen. Es kann somit in vorteilhafter Weise der Schritt umgangen werden, das in der arteriellen Konnektionsnadel befindliche Blut mit Hilfe z.B. einer mit Kochsalzlösung aufgezogenen Spritze retrograd herauszudrücken.

Das erfindungsgemäße Verfahren kann somit den Vorteil bieten, das im Leitungsinneren eines extrakorporalen Blutkreislaufs nach dessen Verwendung bei einer Blutbehandlung vorhandene Blut im Wesentlichen vollständig für den Patienten zurück zu gewinnen.

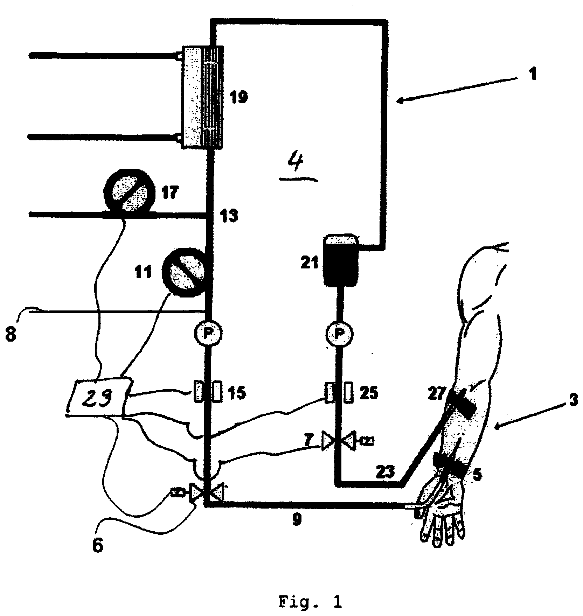

Im Folgenden wird das erfindungsgemäße Verfahren anhand bevorzugter Ausführungsformen desselben unter Bezugnahme auf die Fig. 1 beschrieben.
- Fig. 1: zeigt eine schematische Übersicht einer beispielhaften Anordnung zur Durchführung des erfindungsgemäßen Verfahrens.

In Fig. 1 ist ein extrakorporaler Blutkreislauf 1 mit einem DoubleNeedle-Zugang zum Gefäßsystem des Patienten 3 einer Vorrichtung 4 dargestellt. Der extrakorporale Blutkreislauf 1 wird vom Gefäßsystem des Patienten 3 dekonnektiert, indem eine arterielle Konnektionsnadel 5 aus dem Arm des Patienten gezogen wird.

Zum Starten des erfindungsgemäßen Verfahrens in einer ersten Ausführungsform wird zunächst eine venöse Patientenschlauchklemme 7 geöffnet. Anschließend wird eine Blutpumpe 11 gestartet und so Luft in einen arteriellen Abschnitt 9 des extrakorporalen Blutkreislaufs 1 gesaugt. Die Blutpumpe 11 ist exemplarisch als Rollenpumpe ausgebildet und trägt über das dekonnektierte Ende ein - z.B. vorbestimmtes - Volumen an Luft in den extrakorporalen Blutkreislauf 1 ein. Anschließend wird der Luft-Blut-Inhalt entlang des Leitungsinneren des extrakorporalen Blutkreislaufs 1 durch Betreiben der Blutpumpe 11 in Richtung zu einer venösen Konnektionsnadel 27 gefördert.

Das vorbestimmte Volumen an Luft ist in dieser Ausführungsform so definiert, dass die Luft-Blut-Grenze im Leitungsinneren des extrakorporalen Blutkreislaufs 1 möglichst genau vor der Zugabestelle 13 für Substituatflüssigkeit zum Stehen kommt. Um die Genauigkeit des Stoppens der Luft-Blut-Grenze vor oder an der Zugabestelle 13 für Substituatflüssigkeit zu erhöhen, kann ein arterieller Luft-Blutdetektor 15 an geeigneter Stelle im arteriellen Abschnitt 9 des extrakorporalen Blutkreislaufs 1 zwischen der Zugabestelle 13 für Substituatflüssigkeit und der arteriellen Konnektionsnadel 5 positioniert sein.

Wenn die Luft-Blut-Grenze die Zugabestelle 13 für Substituatflüssigkeit erreicht, wird die Blutpumpe 11 gestoppt. Die zweite Fördereinrichtung, eine hier exemplarisch als Rollenpumpe ausgebildete Substituatpumpe 17, trägt ein - bevorzugt - vorgegebenes Volumen an Substituatflüssigkeit in das Leitungsinnere des extrakorporalen Blutkreislaufs 1 über die Zugabestelle 13 für Substituatflüssigkeit ein. Die Substituatpumpe 17 kann anschließend gestoppt werden.

Alternativ kann die Substituatflüssigkeit auch durch Betreiben der Blutpumpe 11 eingebracht werden. Dazu wird die arterielle Patientenschlauchklemme 6 geschlossen und Substituatflüssigkeit über eine Zuleitung 8 aus einem Vorratsbehälter für die Substituatflüssigkeit in den extrakorporalen Blutkreislauf 1 eingebracht.

Der entstandene Luft-Substituatflüssigkeit-Blut-Inhalt wird durch erneutes Betreiben der Blutpumpe 11 entlang des Leitungsinneren des extrakorporalen Blutkreislaufs 1 gefördert und durch einen Blutfilter 19, eine venöse Luftabscheidekammer 21 und einen venösen Abschnitt 23 des extrakorporalen Blutkreislaufs 1 gedrückt bzw. gefördert, um so das Blut aus dem extrakorporalen Blutkreislauf 1 in Richtung zur venösen Konnektionsnadel 27 zu entfernen. Dabei kann wiederum - durch die Blutpumpe 11 und/oder die Substituatpumpe 17 - Luft eingebracht werden.

In einem venösen Abschnitt 23 des extrakorporalen Blutkreislaufs ist ein venöser Luft-Substituatflüssigkeit-Blutdetektor 25 angeordnet, der das Auftreten von Substituatflüssigkeit an einer vorbestimmten Position des Leitungsinneren des extrakorporalen Blutkreislaufs 1 erkennt. Die Blutpumpe 11 fördert den Luft-Substituatflüssigkeit-Blut-Inhalt solange weiter, bis das Blut im venösen Abschnitt 23 des extrakorporalen Blutkreislaufs aus selbigem entfernt und über die venöse Konnektionsnadel 27 in das Gefäßsystem des Patienten 3 rückgeführt worden ist und/oder bis am venösen Luft-Substituatflüssigkeit-Blutdetektor 25 das Auftreten von Luft im Leitungsinneren erfasst wird. Die Förderleistung aller Pumpen wird beendet. Es kann ein optisches und/oder akustisches Signal ausgegeben werden.

Die Steuerung der Vorrichtung 4 kann mittels einer Steuerungs- oder Regelungseinrichtung 29 erfolgen.

Eine zweite Ausführungsform der vorliegenden Erfindung entspricht im Wesentlichen der vorstehend beschriebenen ersten Ausführungsform, außer dass der Luft-Substituatflüssigkeit-Blut-Inhalt im Leitungsinneren des extrakorporalen Blutkreislaufs 1 nach Zugeben der Substituatflüssigkeit nicht durch Betreiben der Blutpumpe 11, sondern durch Betreiben der Substituatpumpe 17 entlang des Leitungsinneren des extrakorporalen Blutkreislaufs 1 gefördert wird.

Die vorliegende Erfindung ist nicht auf die vorstehend beschriebenen Ausführungsformen beschränkt, diese dienen lediglich der Veranschaulichung. Auch ist die Erfindung nicht auf ein Entleeren des Inhalts oder Teile hiervon bei bestehender Konnektierung mit dem Gefäßsystem beschränkt.

## Patentansprüche

1. Verfahren zum Entfernen von Blut aus einem extrakorporalen Blutkreislauf (1) für eine Behandlungsvorrichtung (4) zur extrakorporalen Blutbehandlung eines Patienten (3) nach Beenden einer Blutbehandlungssitzung,
wobei die Behandlungsvorrichtung (4) umfasst:
- wenigstens einen extrakorporalen Blutkreislauf (1) mit einem Leitungsinneren;
- wenigstens eine Blutpumpe (11) zum Fördern von Blut innerhalb des Leitungsinneren des extrakorporalen Blutkreislaufs (1);
- wenigstens eine Fördereinrichtung (17) zum Einbringen wenigstens einer Substituatflüssigkeit in das Leitungsinnere des extrakorporalen Blutkreislaufs (1) und/oder Fördern eines Leitungsinhalts innerhalb des Leitungsinneren des extrakorporalen Blutkreislaufs (1);
**gekennzeichnet durch** die Schritte:
- Eintragen von Luft in das Leitungsinnere des extrakorporalen Blutkreislaufs (1) **durch** Betreiben der Blutpumpe (11);
- Einbringen von Substituatflüssigkeit in das Leitungsinnere des extrakorporalen Blutkreislaufs (1) **durch** Betreiben einer zweiten Fördereinrichtung (17).

2. Verfahren nach Anspruch 1, wobei die Blutbehandlungsvorrichtung (4) wenigstens eine in einem Abschnitt des extrakorporalen Blutkreislaufs (1) angeordnete Erfassungseinrichtung (15, 25) umfasst, mit dem Schritt:
Erfassen einer qualitativen Änderung des Inhalts des Leitungsinneren des extrakorporalen Blutkreislaufs (1) mittels der Erfassungseinrichtung (15, 25).

3. Verfahren nach einem der vorhergehenden Ansprüche, wobei der extrakorporale Blutkreislauf (1) wenigstens eine mit einem Abschnitt des Gefäßsystems des Patienten (3) konnektierbare Zugangseinrichtung (5) umfasst und das Verfahren ferner den Schritt umfasst:
- Dekonnektieren des extrakorporalen Blutkreislaufs (1) vom Gefäßsystem des Patienten (3).

4. Verfahren nach einem der vorhergehenden Ansprüche, wobei die Blutpumpe (11) ein definiertes Volumen an Luft fördert.

5. Verfahren nach einem der vorhergehenden Ansprüche, wobei ein "Luft-Blut-Inhalt" im Leitungsinneren des extrakorporalen Blutkreislaufs (1) gefördert wird, bis eine "Luft-Blut-Grenze" eine Zugabestelle (13) des extrakorporalen Blutkreislaufs (1) für Substituatflüssigkeit in das Leitungsinnere erreicht, und wobei bei Erreichen eine Substituatflüssigkeit über die Zugabestelle (13) in das Leitungsinnere des extrakorporalen Blutkreislaufs (1) eingebracht wird.

6. Verfahren nach einem der vorhergehenden Ansprüche, wobei durch Betreiben der Fördereinrichtung (17) eine vorbestimmte Substituatflüssigkeitsmenge in das Leitungsinnere des extrakorporalen Blutkreislaufs (1) eingebracht wird.

7. Verfahren nach einem der Ansprüche 2 bis 6, wobei die unter Erzeugen eines "Luft-Substituatflüssigkeit-Blut-Inhalts" in Richtung weg von einem dekonnektierten Ende des extrakorporalen Blutkreislaufs (1)unter Betreiben der Fördereinrichtung (17) zugeführte Substituatflüssigkeit gefördert wird, bis die Erfassungseinrichtung (25) Substituatflüssigkeit im Leitungsinneren des extrakorporalen Blutkreislaufs (1) erfasst.

8. Verfahren nach einem der vorhergehenden Ansprüche, wobei der "Luft-Substituatflüssigkeit-Blut-Inhalt" durch Betreiben der Blutpumpe (11) und/oder durch Betreiben der Fördereinrichtung (17) entlang des Leitungsinneren des extrakorporalen Blutkreislaufs (1) gefördert wird.

9. Verfahren nach einem der vorhergehenden Ansprüche, wobei mittels der Fördereinrichtung (17) zu wenigstens einem ersten Zeitpunkt Substituatflüssigkeit und zu wenigstens einem sich zeitlich vom ersten Zeitpunkt unterscheidenden zweiten Zeitpunkt Luft in das Leitungsinnere des extrakorporalen Blutkreislaufs (1) eingebracht wird.

10. Verfahren nach einem der Ansprüche 2 bis 9, wobei die Erfassungsvorrichtung (25) mit vorgegebener Distanz zu einer zweiten Zugangseinrichtung (27) angeordnet ist und das Verfahren ferner den Schritt umfasst:
- Fördern des Inhalts des Leitungsinneren des extrakorporalen Blutkreislaufs (1) über die vorgegebene Distanz bis zur Zugangseinrichtung (27), nachdem Luft an der Erfassungseinrichtung (25) erkannt wurde.

11. Vorrichtung (4) mit
- wenigstens einem extrakorporalen Blutkreislauf (1) mit einem Leitungsinneren;
- wenigstens einer an oder in dem extrakorporalen Blutkreislauf (1) angeordneten Blutpumpe (11) zum Fördern von Blut innerhalb des Leitungsinneren des extrakorporalen Blutkreislaufs (1);
- wenigstens einer Fördereinrichtung (17) zum Einbringen wenigstens einer Substituatflüssigkeit in das Leitungsinnere des extrakorporalen Blutkreislaufs (1) und/oder Fördern eines Leitungsinhalts innerhalb des Leitungsinneren des extrakorporalen Blutkreislaufs (1);
**gekennzeichnet durch**
- wenigstens eine Steuerungs- und/oder Regelungseinrichtung (29), konfiguriert zum Durchführen des Verfahrens gemäß einem der Ansprüche 1 bis 10.

12. Vorrichtung (4) nach Anspruch 11 mit wenigstens einer in einem Abschnitt des extrakorporalen Blutkreislaufs (1) angeordneten Erfassungseinrichtung (15, 25) zum Erfassen wenigstens einer Änderung des Inhalts oder einer Eigenschaft des Inhalts des Leitungsinneren.

## Claims

1. A method for removing blood from an extracorporeal blood circuit (1) of a treatment apparatus (4) for the extracorporeal blood treatment of a patient (3) following termination of a blood treatment session, wherein the treatment apparatus (4) comprises:
- at least one extracorporeal blood circuit (1) having a conduit interior;
- at least one blood pump (11) for conveying blood inside the conduit interior of the extracorporeal blood circuit (1);
- at least one conveying means (17) for introducing at least one substituate liquid into the conduit interior of the extracorporeal blood circuit (1) and/or for conveying a conduit content inside the conduit interior of the extracorporeal blood circuit (1),
**characterized by** the steps:
- introducing air into the conduit interior of the extracorporeal blood circuit (1) by operating the blood pump (11);
- introducing a substituate liquid into the conduit interior of the extracorporeal blood circuit (1) by operating a second conveying means (17).

2. The method according to claim 1, wherein the treatment apparatus (4) comprises at least one detection means (15, 25) disposed in a portion of the extracorporeal blood circuit (1), with the step:
- detecting a qualitative change of the content of the conduit interior of the extracorporeal blood circuit (1) with the detection means (15, 25).

3. The method according to any one of the preceding claims, wherein the extracorporeal blood circuit (1) comprises at least a first access device (5) adapted to be connected to a portion of the vascular system of the patient (3) and the method further comprises the step:
- disconnecting the extracorporeal blood circuit (1) from the vascular system of the patient (3).

4. The method according to any one of the preceding claims, wherein the blood pump (11) conveys a defined volume of air.

5. The method according to any one of the preceding claims, wherein an "air/blood content" inside the conduit interior of the extracorporeal blood circuit (1) is conveyed until an "air/blood boundary" reaches an addition point (13) for substituate liquid of the extracorporeal blood circuit (1) inside the conduit interior, and wherein, on reaching, a substituate liquid is introduced into the conduit interior of the extracorporeal blood circuit (1) via the addition point (13).

6. The method according to any one of the preceding claims, wherein a predetermined amount of substituate liquid is introduced into the conduit interior of the extracorporeal circuit (1) by operating the conveying means (17).

7. The method according to one of the claims 2 to 6, wherein the substituate liquid introduced by means of an "air/substituate liquid/blood content" in a direction away from a disconnected end portion of the extracorporeal blood circuit (1) by operating the conveying means (17) is conveyed until the detection means (25) detects substituate liquid inside the conduit interior of the extracorporeal blood circuit (1).

8. The method according to any one of the preceding claims, wherein the "air/substituate liquid/blood content" is conveyed along the conduit interior of the extracorporeal blood circuit (1) by operating the blood pump (11) and/or by operating the conveying means (17).

9. The method according to any one of the preceding claims, wherein substituate liquid is introduced into the conduit interior by means of the conveying means (17) at least at a first time point and air is introduced into the conduit interior by means of the conveying means (17) at least at a second time point temporally different from said first time point.

10. The method according to one of claims 2 to 9, wherein the detection means (25) is disposed at a predetermined distance from a second access device (27) and the method further comprises the step:
- conveying the content of the conduit interior of the extracorporeal blood circuit (1) across the predetermined distance to the second access device (27) after air has been detected at the detection means (25).

11. An apparatus (4) comprising:
- at least one extracorporeal blood circuit (1) having a conduit interior;
- at least one blood pump (11) disposed in or at the extracorporeal blood circuit (1) for conveying blood inside the conduit interior of the extracorporeal blood circuit (1);
- at least one second conveying means (17) for introducing at least one substituate liquid into the conduit interior of the extracorporeal blood circuit (1) and/or for conveying a conduit content inside the conduit interior of the extracorporeal blood circuit (1);
**characterized by**
- at least one control and/or regulating means (29) configured for executing the method according to any one of the claims 1 to 10.

12. The apparatus (4) according to claim 11 comprising:
at least one detection means (15, 25) for detecting at least one change of the content or of a characteristic feature of the content of the conduit interior which is disposed in a portion of the extracorporeal blood circuit (1).

## Revendications

1. Un procédé pour enlever le sang d'un circuit sanguin extracorporel (1) d'un appareil de traitement (4) pour le traitement extracorporel du sang d'un patient (3) après la fin d'une séance de traitement du sang, dans lequel l'appareil de traitement (4) comprend:
- au moins un circuit sanguin extracorporel (1) ayant un intérieur de conduit;
- au moins une pompe à sang (11) pour transporter le sang au sein de l'intérieur du conduit du circuit sanguin extracorporel (1);
- au moins un dispositif de transport (17) pour introduire au moins un liquide de substitut dans l'intérieur du conduit du circuit sanguin extracorporel (1) et / ou pour transporter un contenu du conduit au sein de l'intérieur du conduit du circuit sanguin extracorporel (1) ;
**caractérisé par** les étapes consistant à:
- introduire de l'air dans l'intérieur du conduit du circuit sanguin extracorporel (1) au moyen de l'opération de la pompe à sang (11);
- introduire un liquide de substitut dans l'intérieur du conduit du circuit sanguin extracorporel (1) au moyen de l'opération d'un second dispositif de transport (17).

2. Le procédé selon la première revendication, dans lequel l'appareil de traitement du sang (4) comprend au moins un dispositif de détection (15, 25) disposé dans une section du circuit sanguin extracorporel (1),
avec l'étape consistant à:
- détecter un changement qualitatif du contenu de l'intérieur du conduit du circuit sanguin extracorporel (1) au moyen du dispositif de détection (15, 25).

3. Le procédé selon l'une quelconque des revendications précédentes, dans lequel le circuit sanguin extracorporel (1) comprend au moins un dispositif d'accès (5) adapté pour être connecté à une section du système vasculaire du patient (3), le procédé comprenant en outre l'étape consistant à:
- déconnecter le circuit sanguin extracorporel (1) du système vasculaire du patient (3).

4. Le procédé selon l'une quelconque des revendications précédentes, dans lequel la pompe à sang (11) transporte un volume d'air défini.

5. Le procédé selon l'une quelconque des revendications précédentes, dans lequel un contenu « air-sang » à l'intérieur du conduit du circuit sanguin extracorporel (1) est transporté jusqu'à ce qu'une limite « air-sang » atteigne un point d'addition (13) du circuit sanguin extracorporel (1) pour l'addition d'un liquide de substitut dans l'intérieur du conduit et dans lequel un liquide de substitut est introduit via le point d'addition (13) dans l'intérieur du conduit du circuit sanguin extracorporel (1) lorsque la limite air-sang a atteint le point d'addition (13).

6. Le procédé selon l'une quelconque des revendications précédentes, dans lequel une quantité prédéterminée de liquide de substitut est introduite dans l'intérieur du conduit du circuit sanguin extracorporel (1) au moyen de l'opération du dispositif de transport (17).

7. Le procédé selon l'une quelconque des revendications 2 à 6, dans lequel le liquide de substitut introduit sous la création d'un contenu « air - liquide de substitut- sang » est transporté dans une direction opposée à une extrémité déconnectée du circuit sanguin extracorporel (1) au moyen de l'opération du dispositif de transport (17) jusqu'à ce que le dispositif de détection (25) détecte du liquide de substitut à l'intérieur du conduit du circuit sanguin extracorporel (1).

8. Le procédé selon l'une quelconque des revendications précédentes, dans lequel le contenu « air - liquide de substitut- sang » est transporté le long de l'intérieur du conduit du circuit sanguin extracorporel (1) au moyen de l'opération de la pompe à sang (11) et / ou au moyen de l'opération du dispositif de transport (17).

9. Le procédé selon l'une quelconque des revendications précédentes, dans lequel un liquide de substitut est introduit à au moins un premier instant au moyen de l'opération du dispositif de transport (17) dans l'intérieur du conduit du circuit sanguin extracorporel (1) et dans lequel de l'air est introduit à au moins un second instant au moyen de l'opération du dispositif de transport (17) dans l'intérieur du conduit du circuit sanguin extracorporel (1), le second instant différant temporellement du premier instant.

10. Le procédé selon l'une quelconque des revendications 2 à 9, dans lequel le dispositif de détection (25) est disposé à une distance prédéterminée par rapport à un second dispositif d'accès (27), le procédé comprenant en outre l'étape consistant à :
- transporter le contenu de l'intérieur du conduit du circuit sanguin extracorporel (1) sur la distance prédéterminée jusqu'au dispositif d'accès (27) après que de l'air ait été détecté au niveau du dispositif de détection (25).

11. Appareil (4) comprenant:
- au moins un circuit sanguin extracorporel (1) ayant un intérieur de conduit;
- au moins une pompe à sang (11) disposée dans ou au niveau du circuit sanguin extracorporel (1) pour transporter du sang au sein de l'intérieur du conduit du circuit sanguin extracorporel (1) ;
- au moins un second dispositif de transport (17) destiné à introduire au moins un liquide de substitut dans l'intérieur du conduit du circuit sanguin extracorporel (1) et / ou destiné à transporter un contenu du conduit au sein de l'intérieur du conduit du circuit extracorporel de sang (1);
**caractérisé par**
- au moins un dispositif de commande et / ou de réglage (29) configuré pour exécuter un procédé selon l'une quelconque des revendications 1 à 10.

12. L'appareil (4) selon la revendication 11 avec au moins un dispositif de détection (15, 25) pour détecter au moins un changement du contenu ou d'une propriété du contenu de l'intérieur du conduit, le dispositif étant disposé dans une section du circuit sanguin extracorporel (1).
